# EUROPEAN PATENT SPECIFICATION

(11) **EP 2 353 007 B1**
(45) Date of publication and mention of the grant of the patent: **27.01.2016**
(21) Application number: 09764213.6
(22) Date of filing: 11.11.2009
(51) Int. Cl.: G01N 33/564

(54) **ASSESSMENT OF PROTEIN DEGRADATION BY MEASUREMENT OF COLLAGEN FRAGMENTS**
BEURTEILUNG VON PROTEINABBAU DURCH MESSUNG VON COLLAGENFRAGMENTEN
ÉVALUATION DE LA DÉGRADATION DE PROTÉINES PAR MESURE DE FRAGMENTS DE COLLAGÈNE

(30) Priority: 13.11.2008 GB 0820786
(43) Date of publication of application: 10.08.2011
(73) Proprietor: Nordic Bioscience A/S, 2730 Herlev (DK)
(72) Inventor: LEEMING, Diana Julie, S-24 330 Höör (SE); BYRJALSEN, Inger, DK-2970 Hoersholm (DK); QVIST, Per, DK-2930 Klampenborg (DK); KARSDAL, Morten Asser, DK-2100 København Ø (DK)
(74) Representative: Beck Greener
(86) International application number: PCT/EP2009/064997
(87) International publication number: WO 2010/055064

(56) References cited:
- EP-A1- 1 887 358
- WO-A-2004/031725
- US-A1- 2003 219 843
- BYRJALSEN I ET AL: "110 NEW BIOCHEMICAL MARKERS OF COLLAGEN TYPE II DEGRADATION" OSTEOARTHRITIS AND CARTILAGE, BAILLIERE TINDALL, LONDON, GB, vol. 16, 1 September 2008 (2008-09-01), page S60, XP025689824 ISSN: 1063-4584 [retrieved on 2008-09-01]
- OESTERGAARD ET AL: "The utility of measuring C-terminal telopeptides of collagen type II (CTX-II) in serum and synovial fluid samples for estimation of articular cartilage status in experimental models of destructive joint diseases" OSTEOARTHRITIS AND CARTILAGE, BAILLIERE TINDALL, LONDON, GB, vol. 14, no. 7, 1 July 2006 (2006-07-01), pages 670-679, XP005491699 ISSN: 1063-4584
- BILLINGHURST R C ET AL: "Enhanced cleavage of type II collagen by collagenases in ostheoarthritic articular cartilage" JOURNAL OF CLINICAL INVESTIGATION, AMERICAN SOCIETY FOR CLINICAL INVESTIGATION, US, vol. 99, 1 April 1997 (1997-04-01), pages 1534-1545, XP002107397 ISSN: 0021-9738 cited in the application
- DOWNS J T ET AL: "Analysis of collagenase-cleavage of type II collagen using a neoepitope ELISA" JOURNAL OF IMMUNOLOGICAL METHODS, ELSEVIER SCIENCE PUBLISHERS B.V.,AMSTERDAM, NL, vol. 247, no. 1-2, 1 January 2001 (2001-01-01), pages 25-34, XP004228396 ISSN: 0022-1759

### SEQUENCE LISTING

<110> Nordic Bioscience A/S
<120> Assessment of protein degradation by measurement of collagen fragments
<130> PJS/P16429WO
<150> GB 0820786.2
   <151> 2008-11-13
<160> 128
<170> PatentIn version 3.5
<210> 1
   <211> 8
   <212> PRT
   <213> Homo sapiens
<220>
   <221> MISC_FEATURE
   <222> (4)..(4)
   <223> Hydroxyproline
<400> 1
   Glu Gly Pro Pro Gly Pro Gln Gly
   1               5
<210> 2
   <211> 7
   <212> PRT
   <213> Homo sapiens
<220>
   <221> MISC_FEATURE
   <222> (3)..(3)
   <223> Hydroxyproline
<400> 2
   Gly Pro Pro Gly Pro Gln Gly
   1               5
<210> 3
   <211> 7
   <212> PRT
   <213> Homo sapiens
<400> 3
   Gly Pro Pro Gly Pro Gln Gly
   1               5
<210> 4
   <211> 9
   <212> PRT
   <213> Homo sapiens
<400> 4
   Gly Glu Pro Gly Asp Asp Ala Pro Ser
   1 5
<210> 5
   <211> 20
   <212> PRT
   <213> Bos taurus
<400> 5
   Pro Gly Asp Asp Gly Glu Ala Gly Lys Pro Gly Lys Ser Gly Glu Arg
   1 5 10 15
   Gly Pro Pro Gly
   20
<210> 6
   <211> 20
   <212> PRT
   <213> Homo sapiens
<400> 6
   Pro Gly Asp Asp Gly Glu Ala Gly Lys Pro Gly Lys Ala Gly Glu Arg
   1 5 10 15
   Gly Pro Pro Gly
   20
<210> 7
   <211> 7
   <212> PRT
   <213> Homo sapiens
<400> 7
   Gly Pro Pro Gly Pro Gln Gly
   1 5
<210> 8
   <211> 10
   <212> PRT
   <213> Homo sapiens
<400> 8
   Gly Glu Pro Gly Asp Asp Gly Pro Ser Gly
   1 5 10
<210> 9
   <211> 13
   <212> PRT
   <213> Homo sapiens
<400> 9
   Ala Pro Gly Glu Asp Gly Arg Pro Gly Pro Pro Gly Pro
   1               5                   10
<210> 10
   <211> 16
   <212> PRT
   <213> Homo sapiens
<400> 10
   Gly Lys Val Gly Pro Ser Gly Ala Pro Gly Glu Asp Gly Arg Pro Gly
   1               5                   10                  15
<210> 11
   <211> 9
   <212> PRT
   <213> Homo sapiens
<400> 11
   Ala Glu Gly Pro Pro Gly Pro Gln Gly
   1               5
<210> 12
   <211> 10
   <212> PRT
   <213> Homo sapiens
<400> 12
   Gly Pro Pro Gly Pro Gln Gly Leu Ala Gly
   1               5                   10
<210> 13
   <211> 9
   <212> PRT
   <213> Homo sapiens
<400> 13
   Gly Glu Pro Gly Asp Asp Gly Pro Ser
   1               5
<210> 14
   <211> 16
   <212> PRT
   <213> Homo sapiens
<400> 14
   Gly Glu Pro Gly Asp Asp Gly Pro Ser Gly Ala Glu Gly Pro Pro Gly
   1               5                   10                  15
<210> 15
   <211> 21
   <212> PRT
   <213> Homo sapiens
<400> 15
   Glu Lys Gly Glu Pro Gly Asp Asp Ala Pro Ser Gly Ala Glu Gly Pro
   1               5                   10                  15
   Pro Gly Pro Gln Gly
               20
<210> 16
   <211> 21
   <212> PRT
   <213> Homo sapiens
<400> 16
   Gly Pro Pro Gly Pro Pro Gly Lys Pro Gly Asp Asp Gly Glu Ala Gly
   1               5                   10                  15
   Lys Pro Gly Lys Ala
               20
<210> 17
   <211> 21
   <212> PRT
   <213> Homo sapiens
<400> 17
   Gly Pro Pro Gly Pro Arg Gly Arg Ser Gly Glu Thr Gly Pro Ala Gly
   1               5                   10                  15
   Pro Pro Gly Asn Pro
               20
<210> 18
   <211> 22
   <212> PRT
   <213> Homo sapiens
<400> 18
   Gly Ala Pro Gly Pro Gln Gly Phe Gln Gly Asn Pro Gly Glu Pro Gly
   1               5                   10                  15
   Glu Pro Gly Val Ser Tyr
               20
<210> 19
   <211> 20
   <212> PRT
   <213> Homo sapiens
<400> 19
   Gly Glu Pro Gly Asp Asp Ala Gly Pro Ser Gly Ala Glu Gly Pro Pro
   1               5                   10                  15
   Gly Pro Gln Gly
               20
<210> 20
   <211> 7
   <212> PRT
   <213> Homo sapiens
<400> 20
   Gly Glu Ala Gly Lys Pro Gly
   1               5
<210> 21
   <211> 23
   <212> PRT
   <213> Homo sapiens
<400> 21
   Cys Gly Lys Val Gly Pro Ser Gly Ala Pro Gly Glu Asp Gly Arg Pro
   1               5                   10                  15
   Gly Pro Pro Gly Pro Gln Tyr
               20
<210> 22
   <211> 13
   <212> PRT
   <213> Homo sapiens
<400> 22
   Ala Pro Gly Glu Asp Gly Arg Pro Gly Pro Pro Gly Pro
   1               5                   10
<210> 23
   <211> 9
   <212> PRT
   <213> Homo sapiens
<400> 23
   His Arg Gly Tyr Pro Gly Leu Asp Gly
   1               5
<210> 24
   <211> 4
   <212> PRT
   <213> Homo sapiens
<400> 24
   Gly Gln Pro Gly
   1
<210> 25
   <211> 11
   <212> PRT
   <213> Homo sapiens
<400> 25
   Cys Gly Gly Glu Gly Pro Pro Gly Pro Gln Gly
   1 5 10
<210> 26
   <211> 19
   <212> PRT
   <213> Homo sapiens
<400> 26
   Gly Ala Glu Gly Pro Pro Gly Pro Gln Gly Leu Ala Gly Gln Arg Gly
   1 5 10 15
   Ile Val Gly
<210> 27
   <211> 19
   <212> PRT
   <213> Homo sapiens
<400> 27
   Gly Ala Pro Gly Thr Pro Gly Pro Gln Gly Ile Ala Gly Gln Arg Gly
   1 5 10 15
   Val Val Gly
<210> 28
   <211> 19
   <212> PRT
   <213> Homo sapiens
<400> 28
   Gly Pro Pro Gly Thr Pro Gly Pro Gln Gly Leu Leu Gly Ala Pro Gly
   1 5 10 15
   Ile Leu Gly
<210> 29
   <211> 19
   <212> PRT
   <213> Homo sapiens
<400> 29
   Gly Pro Pro Gly Ala Pro Gly Pro Leu Gly Ile Ala Gly Ile Thr Gly
   1 5 10 15
   Ala Arg Gly
<210> 30
   <211> 12
   <212> PRT
   <213> Homo sapiens
<400> 30
   Cys Gly Gly Glu Gly Pro Pro Gly Pro Gln Gly Leu
   1 5 10
<210> 31
   <211> 13
   <212> PRT
   <213> Homo sapiens
<400> 31
   Cys Gly Gly Glu Gly Pro Pro Gly Pro Gln Gly Leu Ala
   1 5 10
<210> 32
   <211> 10
   <212> PRT
   <213> Homo sapiens
<400> 32
   Cys Gly Gly Glu Gly Pro Pro Gly Pro Gln
   1 5 10
<210> 33
   <211> 9
   <212> PRT
   <213> Homo sapiens
<400> 33
   Cys Gly Gly Glu Gly Pro Pro Gly Pro
   1 5
<210> 34
   <211> 8
   <212> PRT
   <213> Homo sapiens
<400> 34
   Cys Gly Pro Pro Gly Pro Gln Gly
   1 5
<210> 35
   <211> 30
   <212> PRT
   <213> Homo sapiens
<400> 35
   Leu Gln Gly Pro Ala Gly Pro Pro Gly Glu Lys Gly Glu Pro Gly Asp
   1               5                   10                  15
   Asp Gly Pro Ser Gly Ala Glu Gly Pro Pro Gly Pro Gln Gly
               20                  25                  30
<210> 36
   <211> 5
   <212> PRT
   <213> Homo sapiens
<400> 36
   Pro Gly Pro Gln Gly
   1               5
<210> 37
   <211> 6
   <212> PRT
   <213> Homo sapiens
<400> 37
   Pro Pro Gly Pro Gln Gly
   1               5
<210> 38
   <211> 31
   <212> PRT
   <213> Homo sapiens
<400> 38
   Val Leu Gln Gly Pro Ala Gly Pro Pro Gly Glu Lys Gly Glu Pro Gly
   1               5                   10                  15
   Asp Asp Gly Pro Ser Gly Ala Glu Gly Pro Pro Gly Pro Gln Gly
               20                  25                  30
<210> 39
   <211> 47
   <212> PRT
   <213> Homo sapiens
<400> 39
   Lys Gly Ala Arg Gly Asp Ser Gly Pro Pro Gly Arg Ala Gly Glu Pro
   1               5                   10                  15
   Gly Leu Gln Gly Pro Ala Gly Pro Pro Gly Glu Lys Gly Glu Pro Gly
               20                  25                  30
   Asp Asp Gly Pro Ser Gly Ala Glu Gly Pro Pro Gly Pro Gln Gly
           35                  40                  45
<210> 40
   <211> 45
   <212> PRT
   <213> Homo sapiens
<400> 40
   Ala Arg Gly Asp Ser Gly Pro Pro Gly Arg Ala Gly Glu Pro Gly Leu
   1               5                   10                  15
   Gln Gly Pro Ala Gly Pro Pro Gly Glu Lys Gly Glu Pro Gly Asp Asp
               20                  25                  30
   Gly Pro Ser Gly Ala Glu Gly Pro Pro Gly Pro Gln Gly
           35                  40                  45
<210> 41
   <211> 28
   <212> PRT
   <213> Homo sapiens
<400> 41
   Gly Pro Ala Gly Pro Pro Gly Glu Lys Gly Glu Pro Gly Asp Asp Gly
   1               5                   10                  15
   Pro Ser Gly Ala Glu Gly Pro Pro Gly Pro Gln Gly
               20                  25
<210> 42
   <211> 145
   <212> PRT
   <213> Homo sapiens
<400> 42
   Gly Pro Ile Gly Pro Pro Gly Glu Arg Gly Ala Pro Gly Asn Arg Gly
   1               5                   10                  15
   Phe Pro Gly Gln Asp Gly Leu Ala Gly Pro Lys Gly Ala Pro Gly Glu
                   20                  25                  30
   Arg Gly Pro Ser Gly Leu Ala Gly Pro Lys Gly Ala Asn Gly Asp Pro
           35                  40                  45
   Gly Arg Pro Gly Glu Pro Gly Leu Pro Gly Ala Arg Gly Leu Thr Gly
       50                  55                  60
   Arg Pro Gly Asp Ala Gly Pro Gln Gly Lys Val Gly Pro Ser Gly Ala
   65                  70                  75                  80
   Pro Gly Glu Asp Gly Arg Pro Gly Pro Pro Gly Pro Gln Gly Ala Arg
                   85                  90                  95
   Gly Gln Pro Gly Val Met Gly Phe Pro Gly Pro Lys Gly Ala Asn Gly
               100                 105                 110
   Glu Pro Gly Lys Ala Gly Glu Lys Gly Leu Pro Gly Ala Pro Gly Leu
           115                 120                 125
   Gly Leu Pro Gly Lys Asp Gly Glu Thr Gly Ala Glu Gly Pro Pro Pro
       130                 135                 140
   Ala
   145
<210> 43
   <211> 347
   <212> PRT
   <213> Homo sapiens
<400> 43
   Met Pro Gly Glu Arg Gly Ala Ala Gly Ile Ala Gly Pro Lys Gly Asp
   1               5                   10                  15
   Arg Gly Asp Val Gly Glu Lys Gly Pro Glu Gly Ala Pro Gly Lys Asp
               20                  25                  30
   Gly Gly Arg Gly Leu Thr Gly Pro Ile Gly Pro Pro Gly Pro Ala Gly
           35                  40                  45
   Ala Asn Gly Glu Lys Gly Glu Val Gly Pro Pro Gly Pro Ala Gly Ser
       50                  55                  60
   Ala Gly Ala Arg Gly Ala Pro Gly Glu Arg Gly Glu Thr Gly Pro Pro
   65                  70                  75                  80
   Gly Thr Ser Gly Ile Ala Gly Pro Pro Gly Ala Asp Gly Gln Pro Gly
   85 90 95
   Ala Lys Gly Glu Gln Gly Glu Ala Gly Gln Lys Gly Asp Ala Gly Ala
   100 105 110
   Pro Gly Pro Gln Gly Pro Ser Gly Ala Pro Gly Pro Gln Gly Pro Thr
   115 120 125
   Gly Val Thr Gly Pro Lys Gly Ala Arg Gly Ala Gln Gly Pro Pro Gly
   130 135 140
   Ala Thr Gly Phe Pro Gly Ala Ala Gly Arg Val Gly Pro Pro Gly Ser
   145 150 155 160
   Asn Gly Asn Pro Gly Pro Pro Gly Pro Pro Gly Pro Ser Gly Lys Asp
   165 170 175
   Gly Pro Lys Gly Ala Arg Gly Asp Ser Gly Pro Pro Gly Arg Ala Gly
   180 185 190
   Glu Pro Gly Leu Gln Gly Pro Ala Gly Pro Pro Gly Glu Lys Gly Glu
   195 200 205
   Pro Gly Asp Asp Gly Pro Ser Gly Ala Glu Gly Pro Pro Gly Pro Gln
   210 215 220
   Gly Leu Ala Gly Gln Arg Gly Ile Val Gly Leu Pro Gly Gln Arg Gly
   225 230 235 240
   Glu Arg Gly Phe Pro Gly Leu Pro Gly Pro Ser Gly Glu Pro Gly Gln
   245 250 255
   Gln Gly Ala Pro Gly Ala Ser Gly Asp Arg Gly Pro Pro Gly Pro Val
   260 265 270
   Gly Pro Pro Gly Leu Thr Gly Pro Ala Gly Glu Pro Gly Arg Glu Gly
   275 280 285
   Ser Pro Gly Ala Asp Gly Pro Pro Gly Arg Asp Gly Ala Ala Gly Val
   290 295 300
   Lys Gly Asp Arg Gly Glu Thr Gly Ala Val Gly Ala Pro Gly Ala Pro
   305 310 315 320
   Gly Pro Pro Gly Ser Pro Gly Pro Ala Gly Pro Thr Gly Lys Gln Gly
   325 330 335
   Asp Arg Gly Glu Ala Gly Ala Gln Gly Pro Met
   340 345
<210> 44
   <211> 6
   <212> PRT
   <213> Homo sapiens
<400> 44
   Glu Lys Gly Pro Asp Pro
   1 5
<210> 45
   <211> 7
   <212> PRT
   <213> Homo sapiens
<220>
   <221> MISC_FEATURE
   <222> (4)..(5)
   <223> Isomerised peptide bond
<400> 45
   Gly Asp Ile Lys Asp Ile Val
   1 5
<210> 46
   <211> 5
   <212> PRT
   <213> Homo sapiens
<220>
   <221> MISC_FEATURE
   <222> (4)..(5)
   <223> Isomerised peptide bond
<400> 46
   Glu Lys Gly Pro Asp
   1 5
<210> 47
   <211> 7
   <212> PRT
   <213> Homo sapiens
<400> 47
   Gly Asp Ile Lys Asp Ile Val
   1 5
<210> 48
   <211> 5
   <212> PRT
   <213> Homo sapiens
<400> 48
   Glu Lys Gly Pro Asp
   1 5
<210> 49
   <211> 5
   <212> PRT
   <213> Homo sapiens
<400> 49
   Pro Gly Val Lys Gly
   1 5
<210> 50
   <211> 6
   <212> PRT
   <213> Homo sapiens
<400> 50
   Pro Gly Pro Lys Gly Glu
   1 5
<210> 51
   <211> 6
   <212> PRT
   <213> Homo sapiens
<400> 51
   Gly Gln Lys Gly Glu Pro
   1 5
<210> 52
   <211> 13
   <212> PRT
   <213> Homo sapiens
<220>
   <221> MISC_FEATURE
   <222> (6)..(6)
   <223> Undefined amino acid
<220>
   <221> MISC_FEATURE
   <222> (8)..(9)
   <223> Undefined amino acids
<220>
   <221> MISC_FEATURE
   <222> (11)..(12)
   <223> Undefined amino acids
<400> 52
   Gly Phe Gln Gly Leu Xaa Gly Xaa Xaa Gly Xaa Xaa Gly
   1 5 10
<210> 53
   <211> 13
   <212> PRT
   <213> Homo sapiens
<220>
   <221> MISC_FEATURE
   <222> (6)..(6)
   <223> undefined amino acid
<220>
   <221> MISC_FEATURE
   <222> (8)..(9)
   <223> undefined amino acids
<220>
   <221> MISC_FEATURE
   <222> (11)..(12)
   <223> undefined amino acids
<400> 53
   Gly Leu Gln Gly Leu Xaa Gly Xaa Xaa Gly Xaa Ser Gly
   1 5 10
<210> 54
   <211> 6
   <212> PRT
   <213> Homo sapiens
<400> 54
   Asp Gln Gly Val Pro Gly
   1 5
<210> 55
   <211> 6
   <212> PRT
   <213> Homo sapiens
<400> 55
   Arg Glu Gly Ser Pro Gly
   1 5
<210> 56
   <211> 6
   <212> PRT
   <213> Homo sapiens
<400> 56
   Leu Ala Gly Pro Lys Gly
   1 5
<210> 57
   <211> 6
   <212> PRT
   <213> Homo sapiens
<400> 57
   Leu Thr Gly Pro Ala Gly
   1               5
<210> 58
   <211> 6
   <212> PRT
   <213> Homo sapiens
<400> 58
   Pro Lys Gly Ala Arg Gly
   1               5
<210> 59
   <211> 6
   <212> PRT
   <213> Homo sapiens
<400> 59
   Gly Gln Pro Gly Pro Ala
   1               5
<210> 60
   <211> 6
   <212> PRT
   <213> Homo sapiens
<400> 60
   Glu Pro Gly Gly Val Gly
   1               5
<210> 61
   <211> 6
   <212> PRT
   <213> Homo sapiens
<400> 61
   Arg Asp Gly Ala Ala Gly
   1               5
<210> 62
   <211> 27
   <212> PRT
   <213> Homo sapiens
<400> 62
   Leu Thr Gly Pro Ala Gly Glu Pro Gly Arg Glu Gly Ser Pro Gly Ala
   1               5                   10                  15
   Asp Gly Pro Pro Gly Arg Asp Gly Ala Ala Gly
               20                  25
<210> 63
   <211> 18
   <212> PRT
   <213> Homo sapiens
<400> 63 Ala Gly
<210> 64
   <211> 16
   <212> PRT
   <213> Homo sapiens
<400> 64
   Ala Gln Gly Pro Pro Gly Ala Thr Gly Phe Pro Gly Ala Ala Gly Arg
   1               5                   10                  15
<210> 65
   <211> 11
   <212> PRT
   <213> Homo sapiens
<400> 65
   Ala Ser Gly Asp Arg Gly Pro Pro Gly Pro Val
   1               5                   10
<210> 66
   <211> 15
   <212> PRT
   <213> Homo sapiens
<400> 66
   Ala Ser Gly Asp Arg Gly Pro Pro Gly Pro Val Gly Pro Pro Gly
   1               5                   10                  15
<210> 67
   <211> 15
   <212> PRT
   <213> Homo sapiens
<400> 67
   Gly Ala Asn Gly Glu Lys Gly Glu Val Gly Pro Pro Gly Pro Ala
   1               5                   10                  15
<210> 68
   <211> 15
   <212> PRT
   <213> Homo sapiens
<400> 68
   Gly Ala Pro Gly Glu Asp Gly Arg Pro Gly Pro Pro Gly Pro Gln
   1 5 10 15
<210> 69
   <211> 18
   <212> PRT
   <213> Homo sapiens
<400> 69 Pro Ala
<210> 70
   <211> 9
   <212> PRT
   <213> Homo sapiens
<400> 70
   Gly Asp Arg Gly Pro Pro Gly Pro Val
   1 5
<210> 71
   <211> 7
   <212> PRT
   <213> Homo sapiens
<400> 71
   Gly Glu Arg Gly Phe Pro Gly
   1 5
<210> 72
   <211> 9
   <212> PRT
   <213> Homo sapiens
<400> 72
   Gly Glu Arg Gly Phe Pro Gly Glu Arg
   1 5
<210> 73
   <211> 15
   <212> PRT
   <213> Homo sapiens
<400> 73
   Gly Glu Ser Gly Ser Pro Gly Glu Asn Gly Ser Pro Gly Pro Met
   1 5 10 15
<210> 74
   <211> 16
   <212> PRT
   <213> Homo sapiens
<400> 74
   Gly Leu Pro Gly Pro Pro Gly Pro Pro Gly Glu Gly Gly Lys Pro Gly
   1 5 10 15
<210> 75
   <211> 9
   <212> PRT
   <213> Homo sapiens
<400> 75
   Gly Pro Ile Gly Pro Pro Gly Pro Ala
   1 5
<210> 76
   <211> 19
   <212> PRT
   <213> Homo sapiens
<400> 76
   Gly Pro Pro Gly Pro Pro Gly Lys Pro Gly Asp Asp Gly Glu Ala Gly
   1 5 10 15
   Lys Pro Gly
<210> 77
   <211> 6
   <212> PRT
   <213> Homo sapiens
<400> 77
   Gly Pro Pro Gly Pro Val
   1 5
<210> 78
   <211> 9
   <212> PRT
   <213> Homo sapiens
<400> 78
   Gly Pro Pro Gly Pro Val Gly Pro Ala
   1 5
<210> 79
   <211> 15
   <212> PRT
   <213> Homo sapiens
<400> 79
   Leu Pro Gly Pro Pro Gly Pro Pro Gly Glu Gly Gly Lys Pro Gly
   1 5 10 15
<210> 80
   <211> 14
   <212> PRT
   <213> Homo sapiens
<400> 80
   Asn Pro Gly Pro Pro Gly Pro Pro Gly Pro Pro Gly Pro Gly
   1 5 10
<210> 81
   <211> 5
   <212> PRT
   <213> Homo sapiens
<400> 81
   Pro Ile Gly Pro Pro
   1 5
<210> 82
   <211> 20
   <212> PRT
   <213> Homo sapiens
<400> 82
   Arg Glu Gly Ser Pro Gly Ala Asp Gly Pro Pro Gly Arg Asp Gly Ala
   1 5 10 15
   Ala Gly Val Lys
   20
<210> 83
   <211> 14
   <212> PRT
   <213> Homo sapiens
<400> 83
   Ser Asn Gly Asn Pro Gly Pro Pro Gly Pro Pro Gly Pro Ser
   1 5 10
<210> 84
   <211> 1060
   <212> PRT
   <213> Homo sapiens
<400> 84
   Gln Met Ala Gly Gly Phe Asp Glu Lys Ala Gly Gly Ala Gln Leu Gly
   1               5                   10                  15
   Val Met Gln Gly Pro Met Gly Pro Met Gly Pro Arg Gly Pro Pro Gly
               20                  25                  30
   Pro Ala Gly Ala Pro Gly Pro Gln Gly Phe Gln Gly Asn Pro Gly Glu
           35                  40                  45
   Pro Gly Glu Pro Gly Val Ser Gly Pro Met Gly Pro Arg Gly Pro Pro
       50                  55                  60
   Gly Pro Pro Gly Lys Pro Gly Asp Asp Gly Glu Ala Gly Lys Pro Gly
   65                  70                  75                  80
   Lys Ala Gly Glu Arg Gly Pro Pro Gly Pro Gln Gly Ala Arg Gly Phe
                   85                  90                  95
   Pro Gly Thr Pro Gly Leu Pro Gly Val Lys Gly His Arg Gly Tyr Pro
               100                 105                 110
   Gly Leu Asp Gly Ala Lys Gly Glu Ala Gly Ala Pro Gly Val Lys Gly
           115                 120                 125
   Glu Ser Gly Ser Pro Gly Glu Asn Gly Ser Pro Gly Pro Met Gly Pro
       130                 135                 140
   Arg Gly Leu Pro Gly Glu Arg Gly Arg Thr Gly Pro Ala Gly Ala Ala
   145                 150                 155                 160
   Gly Ala Arg Gly Asn Asp Gly Gln Pro Gly Pro Ala Gly Pro Pro Gly
                   165                 170                 175
   Pro Val Gly Pro Ala Gly Gly Pro Gly Phe Pro Gly Ala Pro Gly Ala
               180                 185                 190
   Lys Gly Glu Ala Gly Pro Thr Gly Ala Arg Gly Pro Glu Gly Ala Gln
           195                 200                 205
   Gly Pro Arg Gly Glu Pro Gly Thr Pro Gly Ser Pro Gly Pro Ala Gly
       210                 215                 220
   Ala Ser Gly Asn Pro Gly Thr Asp Gly Ile Pro Gly Ala Lys Gly Ser
   225                 230                 235                 240
   Ala Gly Ala Pro Gly Ile Ala Gly Ala Pro Gly Phe Pro Gly Pro Arg
                   245                 250                 255
   Gly Pro Pro Gly Pro Gln Gly Ala Thr Gly Pro Leu Gly Pro Lys Gly
               260                 265                 270
   Gln Thr Gly Glu Pro Gly Ile Ala Gly Phe Lys Gly Glu Gln Gly Pro
           275                 280                 285
   Lys Gly Glu Pro Gly Pro Ala Gly Pro Gln Gly Ala Pro Gly Pro Ala
       290                 295                 300
   Gly Glu Glu Gly Lys Arg Gly Ala Arg Gly Glu Pro Gly Gly Val Gly
   305                 310                 315                 320
   Pro Ile Gly Pro Pro Gly Glu Arg Gly Ala Pro Gly Asn Arg Gly Phe
                   325                 330                 335
   Pro Gly Gln Asp Gly Leu Ala Gly Pro Lys Gly Ala Pro Gly Glu Arg
               340                 345                 350
   Gly Pro Ser Gly Leu Ala Gly Pro Lys Gly Ala Asn Gly Asp Pro Gly
           355                 360                 365
   Arg Pro Gly Glu Pro Gly Leu Pro Gly Ala Arg Gly Leu Thr Gly Arg
       370                 375                 380
   Pro Gly Asp Ala Gly Pro Gln Gly Lys Val Gly Pro Ser Gly Ala Pro
   385                 390                 395                 400
   Gly Glu Asp Gly Arg Pro Gly Pro Pro Gly Pro Gln Gly Ala Arg Gly
                   405                 410                 415
   Gln Pro Gly Val Met Gly Phe Pro Gly Pro Lys Gly Ala Asn Gly Glu
               420                 425                 430
   Pro Gly Lys Ala Gly Glu Lys Gly Leu Pro Gly Ala Pro Gly Leu Arg
           435                 440                 445
   Gly Leu Pro Gly Lys Asp Gly Glu Thr Gly Ala Ala Gly Pro Pro Gly
       450                 455                 460
   Pro Ala Gly Pro Ala Gly Glu Arg Gly Glu Gln Gly Ala Pro Gly Pro
   465 470 475 480
   Ser Gly Phe Gln Gly Leu Pro Gly Pro Pro Gly Pro Pro Gly Glu Gly
   485 490 495
   Gly Lys Pro Gly Asp Gln Gly Val Pro Gly Glu Ala Gly Ala Pro Gly
   500 505 510
   Leu Val Gly Pro Arg Gly Glu Arg Gly Phe Pro Gly Glu Arg Gly Ser
   515 520 525
   Pro Gly Ala Gln Gly Leu Gln Gly Pro Arg Gly Leu Pro Gly Thr Pro
   530 535 540
   Gly Thr Asp Gly Pro Lys Gly Ala Ser Gly Pro Ala Gly Pro Pro Gly
   545 550 555 560
   Ala Gln Gly Pro Pro Gly Leu Gln Gly Met Pro Gly Glu Arg Gly Ala
   565 570 575
   Ala Gly Ile Ala Gly Pro Lys Gly Asp Arg Gly Asp Val Gly Glu Lys
   580 585 590
   Gly Pro Glu Gly Ala Pro Gly Lys Asp Gly Gly Arg Gly Leu Thr Gly
   595 600 605
   Pro Ile Gly Pro Pro Gly Pro Ala Gly Ala Asn Gly Glu Lys Gly Glu
   610 615 620
   Val Gly Pro Pro Gly Pro Ala Gly Ser Ala Gly Ala Arg Gly Ala Pro
   625 630 635 640
   Gly Glu Arg Gly Glu Thr Gly Pro Pro Gly Pro Ala Gly Phe Ala Gly
   645 650 655
   Pro Pro Gly Ala Asp Gly Gln Pro Gly Ala Lys Gly Glu Gln Gly Glu
   660 665 670
   Ala Gly Gln Lys Gly Asp Ala Gly Ala Pro Gly Pro Gln Gly Pro Ser
   675 680 685
   Gly Ala Pro Gly Pro Gln Gly Pro Thr Gly Val Thr Gly Pro Lys Gly
   690 695 700
   Ala Arg Gly Ala Gln Gly Pro Pro Gly Ala Thr Gly Phe Pro Gly Ala
   705 710 715 720
   Ala Gly Arg Val Gly Pro Pro Gly Ser Asn Gly Asn Pro Gly Pro Pro
                   725                 730                 735
   Gly Pro Pro Gly Pro Ser Gly Lys Asp Gly Pro Lys Gly Ala Arg Gly
               740                 745                 750
   Asp Ser Gly Pro Pro Gly Arg Ala Gly Glu Pro Gly Leu Gln Gly Pro
           755                 760                 765
   Ala Gly Pro Pro Gly Glu Lys Gly Glu Pro Gly Asp Asp Gly Pro Ser
       770                 775                 780
   Gly Ala Glu Gly Pro Pro Gly Pro Gln Gly Leu Ala Gly Gln Arg Gly
   785                 790                 795                 800
   Ile Val Gly Leu Pro Gly Gln Arg Gly Glu Arg Gly Phe Pro Gly Leu
                   805                 810                 815
   Pro Gly Pro Ser Gly Glu Pro Gly Lys Gln Gly Ala Pro Gly Ala Ser
               820                 825                 830
   Gly Asp Arg Gly Pro Pro Gly Pro Val Gly Pro Pro Gly Leu Thr Gly
           835                 840                 845
   Pro Ala Gly Glu Pro Gly Arg Glu Gly Ser Pro Gly Ala Asp Gly Pro
       850                 855                 860
   Pro Gly Arg Asp Gly Ala Ala Gly Val Lys Gly Asp Arg Gly Glu Thr
   865                 870                 875                 880
   Gly Ala Val Gly Ala Pro Gly Ala Pro Gly Pro Pro Gly Ser Pro Gly
                   885                 890                 895
   Pro Ala Gly Pro Thr Gly Lys Gln Gly Asp Arg Gly Glu Ala Gly Ala
               900                 905                 910
   Gln Gly Pro Met Gly Pro Ser Gly Pro Ala Gly Ala Arg Gly Ile Gln
           915                 920                 925
   Gly Pro Gln Gly Pro Arg Gly Asp Lys Gly Glu Ala Gly Glu Pro Gly
       930                 935                 940
   Glu Arg Gly Leu Lys Gly His Arg Gly Phe Thr Gly Leu Gln Gly Leu
   945                 950                 955                 960
   Pro Gly Pro Pro Gly Pro Ser Gly Asp Gln Gly Ala Ser Gly Pro Ala
   965 970 975
   Gly Pro Ser Gly Pro Arg Gly Pro Pro Gly Pro Val Gly Pro Ser Gly
   980 985 990
   Lys Asp Gly Ala Asn Gly Ile Pro Gly Pro Ile Gly Pro Pro Gly Pro
   995 1000 1005
   Arg Gly Arg Ser Gly Glu Thr Gly Pro Ala Gly Pro Pro Gly Asn
   1010 1015 1020
   Pro Gly Pro Pro Gly Pro Pro Gly Pro Pro Gly Pro Gly Ile Asp
   1025 1030 1035
   Met Ser Ala Phe Ala Gly Leu Gly Pro Arg Glu Lys Gly Pro Asp
   1040 1045 1050
   Pro Leu Gln Tyr Met Arg Ala
   1055 1060
<210> 85
   <211> 23
   <212> PRT
   <213> Homo sapiens
<400> 85
   Ala Ala Gly Ala Arg Gly Asn Asp Gly Gln Pro Gly Pro Ala Gly Pro
   1 5 10 15
   Pro Gly Pro Val Gly Pro Ala
   20
<210> 86
   <211> 18
   <212> PRT
   <213> Homo sapiens
<400> 86
   Ala Gln Gly Pro Arg Gly Glu Pro Gly Thr Pro Gly Ser Pro Gly Pro
   1 5 10 15
   Ala Gly
<210> 87
   <211> 18
   <212> PRT
   <213> Homo sapiens
<400> 87
   Ala Arg Gly Ala Pro Gly Glu Arg Gly Glu Thr Gly Pro Pro Gly Pro
   1 5 10 15
   Ala Gly
<210> 88
   <211> 11
   <212> PRT
   <213> Homo sapiens
<400> 88
   Ala Ser Gly Asp Arg Gly Pro Pro Gly Pro Val
   1 5 10
<210> 89
   <211> 12
   <212> PRT
   <213> Homo sapiens
<400> 89
   Ala Ser Gly Asp Arg Gly Pro Pro Gly Pro Val Gly
   1 5 10
<210> 90
   <211> 9
   <212> PRT
   <213> Homo sapiens
<400> 90
   Ala Thr Gly Pro Leu Gly Pro Lys Gly
   1 5
<210> 91
   <211> 12
   <212> PRT
   <213> Homo sapiens
<400> 91
   Asp Arg Gly Pro Pro Gly Pro Val Gly Pro Pro Gly
   1 5 10
<210> 92
   <211> 30
   <212> PRT
   <213> Homo sapiens
<400> 92
   Glu Arg Gly Ala Pro Gly Asn Arg Gly Phe Pro Gly Gln Asp Gly Leu
   1 5 10 15
   Ala Gly Pro Lys Gly Ala Pro Gly Glu Arg Gly Pro Ser Gly
   20 25 30
<210> 93
   <211> 35
   <212> PRT
   <213> Homo sapiens
<400> 93
   Phe Gln Gly Leu Pro Gly Pro Pro Gly Pro Pro Gly Glu Gly Gly Lys
   1               5                   10                  15
   Pro Gly Asp Gln Gly Val Pro Gly Glu Ala Gly Ala Pro Gly Leu Val
               20                  25                  30
   Gly Pro Arg
           35
<210> 94
   <211> 36
   <212> PRT
   <213> Homo sapiens
<400> 94
   Phe Gln Gly Leu Pro Gly Pro Pro Gly Pro Pro Gly Glu Gly Gly Lys
   1               5                   10                  15
   Pro Gly Asp Gln Gly Val Pro Gly Glu Ala Gly Ala Pro Gly Leu Val
               20                  25                  30
   Gly Pro Arg Gly
           35
<210> 95
   <211> 15
   <212> PRT
   <213> Homo sapiens
<400> 95
   Phe Thr Gly Leu Gln Gly Leu Pro Gly Pro Pro Gly Pro Ser Gly
   1               5                   10                  15
<210> 96
   <211> 39
   <212> PRT
   <213> Homo sapiens
<400> 96
   Phe Thr Gly Leu Gln Gly Leu Pro Gly Pro Pro Gly Pro Ser Gly Asp
   1               5                   10                  15
   Gln Gly Ala Ser Gly Pro Ala Gly Pro Ser Gly Pro Arg Gly Pro Pro
   20 25 30
   Gly Pro Val Gly Pro Ser Gly
   35
<210> 97
   <211> 15
   <212> PRT
   <213> Homo sapiens
<400> 97
   Gly Ala Asn Gly Glu Lys Gly Glu Val Gly Pro Pro Gly Pro Ala
   1 5 10 15
<210> 98
   <211> 15
   <212> PRT
   <213> Homo sapiens
<400> 98
   Gly Ala Pro Gly Glu Asp Gly Arg Pro Gly Pro Pro Gly Pro Gln
   1 5 10 15
<210> 99
   <211> 16
   <212> PRT
   <213> Homo sapiens
<400> 99
   Gly Ala Pro Gly Glu Asp Gly Arg Pro Gly Pro Pro Gly Pro Gln Gly
   1 5 10 15
<210> 100
   <211> 15
   <212> PRT
   <213> Homo sapiens
<400> 100
   Gly Ala Pro Gly Glu Arg Gly Glu Thr Gly Pro Pro Gly Pro Ala
   1 5 10 15
<210> 101
   <211> 15
   <212> PRT
   <213> Homo sapiens
<400> 101
   Gly Glu Ser Gly Ser Pro Gly Glu Asn Gly Ser Pro Gly Pro Met
   1 5 10 15
<210> 102
   <211> 9
   <212> PRT
   <213> Homo sapiens
<400> 102
   Gly Pro Ile Gly Pro Pro Gly Pro Ala
   1               5
<210> 103
   <211> 6
   <212> PRT
   <213> Homo sapiens
<400> 103
   Gly Pro Pro Gly Pro Val
   1               5
<210> 104
   <211> 10
   <212> PRT
   <213> Homo sapiens
<400> 104
   Gly Pro Pro Gly Pro Val Gly Pro Pro Gly
   1               5                   10
<210> 105
   <211> 16
   <212> PRT
   <213> Homo sapiens
<400> 105
   Gly Pro Arg Gly Pro Pro Gly Pro Ala Gly Ala Pro Gly Pro Gln Gly
   1               5                   10                  15
<210> 106
   <211> 26
   <212> PRT
   <213> Homo sapiens
<400> 106
   Gly Val Met Gln Gly Pro Met Gly Pro Met Gly Pro Arg Gly Pro Pro
   1               5                   10                  15
   Gly Pro Ala Gly Ala Pro Gly Pro Gln Gly
               20                  25
<210> 107
   <211> 25
   <212> PRT
   <213> Homo sapiens
<400> 107
   Ile Val Gly Leu Pro Gly Gln Arg Gly Glu Arg Gly Phe Pro Gly Leu
   1               5                   10                  15
   Pro Gly Pro Ser Gly Glu Pro Gly Lys
               20                  25
<210> 108
   <211> 21
   <212> PRT
   <213> Homo sapiens
<400> 108
   Lys Gln Gly Asp Arg Gly Glu Ala Gly Ala Gln Gly Pro Met Gly Pro
   1               5                   10                  15
   Ser Gly Pro Ala Gly
               20
<210> 109
   <211> 21
   <212> PRT
   <213> Homo sapiens
<400> 109
   Lys Val Gly Pro Ser Gly Ala Pro Gly Glu Asp Gly Arg Pro Gly Pro
   1               5                   10                  15
   Pro Gly Pro Gln Gly
               20
<210> 110
   <211> 21
   <212> PRT
   <213> Homo sapiens
<400> 110
   Leu Pro Gly Lys Asp Gly Glu Thr Gly Ala Ala Gly Pro Pro Gly Pro
   1               5                   10                  15
   Ala Gly Pro Ala Gly
               20
<210> 111
   <211> 33
   <212> PRT
   <213> Homo sapiens
<400> 111
   Leu Pro Gly Lys Asp Gly Glu Thr Gly Ala Ala Gly Pro Pro Gly Pro
   1               5                   10                  15
   Ala Gly Pro Ala Gly Glu Arg Gly Glu Gln Gly Ala Pro Gly Pro Ser
               20                  25                  30
   Gly
<210> 112
   <211> 36
   <212> PRT
   <213> Homo sapiens
<400> 112
   Leu Gln Gly Leu Pro Gly Pro Pro Gly Pro Ser Gly Asp Gln Gly Ala
   1               5                   10                  15
   Ser Gly Pro Ala Gly Pro Ser Gly Pro Arg Gly Pro Pro Gly Pro Val
               20                  25                  30
   Gly Pro Ser Gly
           35
<210> 113
   <211> 17
   <212> PRT
   <213> Homo sapiens
<400> 113
   Leu Thr Gly Pro Ala Gly Glu Pro Gly Arg Glu Gly Ser Pro Gly Ala
   1               5                   10                  15
   Asp
<210> 114
   <211> 10
   <212> PRT
   <213> Homo sapiens
<400> 114
   Gly Pro Pro Gly Arg Asp Gly Ala Ala Gly
   1               5                   10
<210> 115
   <211> 12
   <212> PRT
   <213> Homo sapiens
<400> 115
   Leu Thr Gly Pro Ile Gly Pro Pro Gly Pro Ala Gly
   1 5 10
<210> 116
   <211> 33
   <212> PRT
   <213> Homo sapiens
<400> 116
   Leu Thr Gly Arg Pro Gly Asp Ala Gly Pro Gln Gly Lys Val Gly Pro
   1 5 10 15
   Ser Gly Ala Pro Gly Glu Asp Gly Arg Pro Gly Pro Pro Gly Pro Gln
   20 25 30
   Gly
<210> 117
   <211> 23
   <212> PRT
   <213> Homo sapiens
<400> 117
   Gln Gly Pro Met Gly Pro Met Gly Pro Arg Gly Pro Pro Gly Pro Ala
   1 5 10 15
   Gly Ala Pro Gly Pro Gln Gly
   20
<210> 118
   <211> 31
   <212> PRT
   <213> Homo sapiens
<400> 118
   Gln Gly Pro Arg Gly Leu Pro Gly Thr Pro Gly Thr Asp Gly Pro Lys
   1 5 10 15
   Gly Ala Ser Gly Pro Ala Gly Pro Pro Gly Ala Gln Gly Pro Pro
   20 25 30
<210> 119
   <211> 28
   <212> PRT
   <213> Homo sapiens
<400> 119
   Arg Ser Gly Glu Thr Gly Pro Ala Gly Pro Pro Gly Asn Pro Gly Pro
   1               5                   10                  15
   Pro Gly Pro Pro Gly Pro Pro Gly Pro Gly Ile Asp
               20                  25
<210> 120
   <211> 21
   <212> PRT
   <213> Homo sapiens
<400> 120
   Arg Val Gly Pro Pro Gly Ser Asn Gly Asn Pro Gly Pro Pro Gly Pro
   1               5                   10                  15
   Pro Gly Pro Ser Gly
               20
<210> 121
   <211> 14
   <212> PRT
   <213> Homo sapiens
<400> 121
   Ser Asn Gly Asn Pro Gly Pro Pro Gly Pro Pro Gly Pro Ser
   1               5                   10
<210> 122
   <211> 9
   <212> PRT
   <213> Homo sapiens
<400> 122
   Ser Pro Gly Pro Met Gly Pro Arg Gly
   1               5
<210> 123
   <211> 21
   <212> PRT
   <213> Homo sapiens
<400> 123
   Val Lys Gly Glu Ser Gly Ser Pro Gly Glu Asn Gly Ser Pro Gly Pro
   1               5                   10                  15
   Met Gly Pro Arg Gly
               20
<210> 124
   <211> 10
   <212> PRT
   <213> Homo sapiens
<400> 124
   Leu Thr Gly Pro Ala Gly Gly Gly Gly Cys
   1               5                   10
<210> 125
   <211> 10
   <212> PRT
   <213> Homo sapiens
<400> 125
   Leu Thr Gly Pro Ala Gly Glu Pro Gly Lys
   1               5                   10
<210> 126
   <211> 11
   <212> PRT
   <213> Homo sapiens
<400> 126
   Gly Leu Thr Gly Pro Ala Gly Glu Pro Gly Lys
   1               5                   10
<210> 127
   <211> 10
   <212> PRT
   <213> Homo sapiens
<400> 127
   Lys Gly Ala Thr Gly Pro Leu Gly Pro Lys
   1               5                   10
<210> 128
   <211> 10
   <212> PRT
   <213> Homo sapiens
<400> 128
   Cys Gly Gly Gly Arg Asp Gly Ala Ala Gly
   1               5                   10

## Description

The present invention relates to the measurements in body fluids of certain peptide fragments generated by proteolytic cleavage of type II collagen using two antibodies each recognising a neo-epitope located on said peptide fragment for quantitative assessment of articular cartilage degradation.

Osteoarthrtis is one of the leading causes of disability in the world, with more than 10% of the elderly population having symptomatic disease (Woolf & Pfleger, 2003). The incidence increases with age, and by age 65, 80% has radiographic evidence of OA (Lawrence et al., 1998). Therefore, osteoarthritis is both prevalent and a serious burden to the patient and the society. However, at present there is little to offer the affected individuals for prevention of the disease or treatment in the early stages. For many patients, hip or knee replacement is eventually the only treatment option.

Although the pathogenicity of osteoarthritis is not fully understood at present, it is evident that a central hallmark in this slow, chronic disease is progressive destruction of the articular joints, which consist of bone, cartilage and the synovium. In particular the cartilage has attracted much attention, and the different grades and stages of OA cartilage histopathology have recently been detailed described by a working group under OARSI (Pritzker et al., 2006). This system encompasses 7 grades (or severity levels) with involvement of the deeper cartilage layers in more advanced OA disease, and when combined with the extent of cartilage involvement expressed in 5 stages this leads to a semi-quantitative scoring system of 0 to 24.

However, the lack of sensitive, specific and fast analytical techniques to assess important metabolic processes in articular cartilage and their effects on the structure of the tissue is a major barriere to effective drug development in OA.

In particular, major efforts have been allocated to the development of new and better biochemical markers of cartilage turnover.

Cartilage, including articular cartilage, is for the most part composed of collagen type II (60%-70% of dry weight) and proteoglycans (10% of dry weight). Cartilage degradation is mainly mediated by the MMPs and the closely related ADAM-TS (a disintegrin and metalloproteinase with thrombospondin motifs), but collagen type II is most sensitive to MMP activity (Dean et al., 1989; Reboul et al., 1996; Hui et al., 2003). The action of these proteases results in the release of various extracellular fragments which could be candidates as biomarkers of cartilage degradation.

Proteolytic cleavage of type II collagen has been reported in numerous studies, and several different degradation fragments of collagen type II have been indicated as useful for monitoring degenerative diseases of the cartilage (Schaller et al., 2005; , Sumer et al., 2006; Birmingham et al., 2006).

A fragment of the C-telopeptide of type II collagen, i.e. CTX-II, is generated by MMP- activity (Christgau et al., 2001; Mouritzen et al., 2003) and measurement of CTX-II has been reported for monitoring degradation of type II collagen in experimental setups assessing cartilage degradation (Schaller et al., 2005) as well as in humans (Reijman et al., 2004).

However, the first report of using antibodies for detection of collagen type II fragments came from Billinghurst and co-workers (Billinghurst et al., 1997), who described detection of an amino-terminal neoepitope on the shorter fragment of type II collagen after cleavage by collagenase.

More well-described in the literature is the C2C neoepitope at the C-terminus of the ¾ length fragment (Fraser et al., 2003; Poole et al., 2004). This test is dependant of the binding of a monoclonal antibody to the amino acid sequence EGPP(OH)GPQG SEQ ID NO:1 (Poole et al., 2004).

Other tests for collagen type II fragments include the C1,2C, which, however, is based on the amino acid sequence GPP(OH)GPQG SEQ ID NO:2 found in both type I and type II (Billinghurts et al., 1997). Also, another fragment generated by the action of collagenase is the TIINE fragment (Otterness et al., 1997) and can be detected using monoclonal antibody 9A4 recognising the neoepitope Gly-Pro-Pro-Gly-Pro-Gln-Gly-COOH SEQ ID NO:3. Combined with monoclonal antibody 5109 (Downs et al., 2001) as a capture antibody, the sandwich test is claimed to be specific for type II collagen fragments. Monoclonal antibody 5109 binds to the internal amino acid sequence GEPGDDAPS SEQ ID NO:20 and the sandwich test detects collagen fragments of variable length at the N-terminus of the binding sequence of monoclonal antibody 5109.

Zhen (2008) and co-workers discloses identification and characterization of proteolytic peptide products of human articular cartilage, including type II collagen fragments.

Cathepsin K cleavage of type II collagen has been reported in a few studies. The first study to associate cathepsin K with cleavage of type II collagen was reported by Kafienah et al. (1998). Kafienah and coworkers described helical cleavage site(s) of collagen type II by Cathepsin K, and provided the amino acid sequence for one cleavage site, i.e.
PGDDGEAGKPG⇓KSGERGPPG (bovine sequence) SEQ ID NO:5

Ten years after, Dejica et al. (2008) reported the development of an enzyme-linked immunosorbent assay based on polyclonal antibodies against the C-terminal neoepitope (C2K) of the cathepsin K cleavage site reported by Kefienah, i.e.
PGDDGEAGKPG⇓KAGERGPPG SEQ ID NO:6
As the seven C-terminal amino acids of this epitope, i.e. GEAGKPG SEQ ID NO:20 can be found in type I collagen as well, this test will not distinguish type I and type II collagen fragments generated by cathepsin K activity. In contrast, according to the present invention such sequences are deselected to increase specificity for type II collagen fragments.

U.S. Pat. No. 6,642,007 (Saltarelli) disclose methods for monitoring urine for type II collagen fragment using a combination of a capture antibody and a detection antibody, such that type II collagen is distinguished from other collagen fragments.

U.S. Pat. No. 6,030,792 (Otterness) discloses antibodies for detecting collagen type II fragments resulting from collagenase cleavage. In particular, the following sequences are disclosed;
o GPPGPQG SEQ ID NO:7
o GEPGDDGPSG SEQ ID NO:8
o APGEDGRPGPPGP SEQ ID NO:9
o GKVGPSGAPGEDGRPG SEQ ID NO:10
o AEGPPGPQG SEQ ID NO:11
o GPPGPQGLAG SEQ ID NO:12
o GEPGDDGPS SEQ ID NO:13
o GEPGDDGPSGAEGPPG SEQ ID NO:14
o EKGEPGDDAPSGAEGPPGPQG SEQ ID NO:15
o GPPGPPGKPGDDGEAGKPGKA SEQ ID NO:16
o GPPGPRGRSGETGPAGPPGNP SEQ ID NO:17
o GAPGPQGFQGNPGEPGEPGVSY SEQ ID NO: 18
o GEPGDDAGPSGAEGPPGPQG SEQ ID NO: 19

A series of patents (US Pat. No. 6,602,980; 6,566,492; 6,348,320; 6,255,056; 6,153,732; 6,143,511; 6,100,379; 5,919,634; 5,702,909; 5, 688, 652; 5, 641, 837; 5, 641, 687; 5,532,169) (Eyre) relates to peptides and methods for cartilage resorption assays employing antibodies binding to epitopes in the telopeptides of type II collagen.

US Pat. No. 5,283,197 (Robins) describes methods of detecting collagen fragments cross-linked with lysyl pyridinoline or hydroxylysyl pyridinoline.

US Pat. No. 7,410,770 (Reginster) disclosed methods for detection of collagenase-generated fragments of collagen type II using antibody binding to epitope in the amino acid sequence HRGYPGLDG SEQ ID NO: 23 located in the helical region of collagen type II.

US Pat. No. 6,132,976 (Poole) discloses methods for detecting cartilage degradation using antibodies which does not bind to unwound (native) type II collagen fragments but only to fragments being generated by collagenase cleavage. In particular the following amino acid sequences originating from type II collagen are included;
∘ CGKVGPSGAPGEDGRPGPPGPQY SEQ ID NO: 21
∘ APGEDGRPGPPGP SEQ ID NO: 22
∘ GQPG SEQ ID NO: 24
∘ GPPGPQG SEQ ID NO: 7
∘ CGGEGPPGPQG SEQ ID NO: 25
∘ GAEGPPGPQGLAGQRGIVG SEQ ID NO: 26
∘ GAPGTPGPQGIAGQRGVVG SEQ ID NO: 27
∘ GPPGTPGPQGLLGAPGILG SEQ ID NO: 28
∘ GPPGAPGPLGIAGITGARG SEQ ID NO: 29
∘ CGGEGPPGPQGL SEQ ID NO: 30
∘ CGGEGPPGPQGLA SEQ ID NO: 31
∘ CGGEGPPGPQ SEQ ID NO: 32
∘ CGGEGPPGP SEQ ID NO: 33
∘ CGPPGPQG SEQ ID NO: 34

US Pat. No. 7,115,378 (Welsch) describes the use of mass spectrometry for identifying and quantifying peptides resulting from enzyme cleavage of collagen type II. The technique is used for identification and quantification of the peptides in a biological sample to assess activity of proteolytic enzymes in osteoarthritis and rheumatoid arthritis. In particular, the following sequences originating from humans are disclosed;
∘ GPPGPQG SEQ ID NO:7
∘ LQGPAGPPGEKGEPGDDGPSGAEGPPGPQG SEQ ID NO:35
∘ PQG
∘ PGPQG SEQ ID NO:36
∘ PPGPQG SEQ ID NO:37
∘ VLQGPAGPPGEKGEPGDDGPSGAEGPPGPQG SEQ ID NO:38
∘ KGARGDSGPPGRAGEPGLQGPAGPPGEKGEPGDDGPSGAEGPPGPQG SEQ ID NO:39
∘ ARGDSGPPGRAGEPGLQGPAGPPGEKGEPGDDGPSGAEGPPGPQG SEQ ID NO:40
∘ GPAGPPGEKGEPGDDGPSGAEGPPGPQG SEQ ID NO:41
∘

US Pat. No. 6,706,490 (Cook) describes the detection of antibodies to collagen using CB peptides, in particular CB10, of mammalian type II collagen. Cyanogen bromide cleaves the carboxyl terminal of methionine residues thereby producing the CB peptides. The CB10 peptide has the sequence:
∘
∘ US Pat. No. 7,195,883 (Rosenquist) disclose sandwich immunoassays in which a single antibody specific for the amino acid sequence EKGPDP SEQ ID NO:44 is used to detect telopeptide fragments of type II collagen.

US Pat. No. 6,420,125 and 6,107,047 (Fledelius) disclose methods of measuring the rate of degradation of collagen using antibodies binding to an amino acid sequence of type II collagen containing an isoaspartic acid residue. Also, the use of synthetic peptides having an amino acid sequence of type II collagen that contains an isoaspartic acid residue is described. In particular, the following sequences from type II collagen are described;
∘ GDIK*DIV SEQ ID NO:45
∘ EKGP*D SEQ ID NO:46, where (*) denotes an isomerised peptide bond.

US Pat. No. 6,300,083 (Fledelius) describes the determination of the amount of a D-amino acid containing fragment of the protein in a body fluid using an antibody capable of discriminating between the D-amino acid containing fragment and its L-amino acid containing analogue. In particular, the application includes the following peptide sequences from type II collagen;
∘ GDIKDIV SEQ ID NO:47
∘ EKGPD SEQ ID NO:48

US Pat. No. 6,372,442 and 6,210,902 (Bonde) describes methods of characterizing the degradation of type II collagen. At least two distinct immunological assays should be used, each using a different immunological binding partner, and a numerical index is formed representing the difference in the results of the assays. In particular, the following type II collagen sequences are disclosed;
∘ EKGPDP SEQ ID NO:44
∘ EKGPD SEQ ID NO:48
∘ GVK
∘ PGVKG SEQ ID NO:49
∘ PGPKGE SEQ ID NO:50
∘ GQKGEP SEQ ID NO:51
∘ GDIKDIV SEQ ID NO:47

US Pat. No. 6,355,442; 6,342,361; 6,323,314 and 6,110,689 (Qvist) describe the use of antibodies recognizing synthetic peptides for detection of collagen fragments. In particular, the following amino acid sequences are included;
∘ PGPKGE SEQ ID NO:50
∘ GQKGEP SEQ ID NO:51
∘ GDIKDIV SEQ ID NO:47
∘ EKGPD SEQ ID NO:48
∘ GVK
∘ PGVKG SEQ ID NO:49

US Pat. No. 6,010,863 (Te Koppele) discloses the use of a sandwich immunoassay for the detection of collagen degradation using a first antibody directed at an epitope present on a collagen molecule at a distance of up to 165 amino acids from a collagen telopeptide crosslink site, and a second antibody directed at another epitope of the crosslinked collagen molecule.

US Pat. No. 5,541,295 (Barrach) discloses monoclonal antibodies which bind specifically to Type II collagen, but not to its peptides, or vice versa. In particular, the following type II collagen sequences are included;
- GFQGL-Xaa-G-Xaa-Xaa-G-Xaa-Xaa-G SEQ ID NO:52
- GLQGL-Xaa-G-Xaa-Xaa-G-Xaa-SG SEQ ID NO:53

None of the above mentioned patents disclose the use of two antibodies each of which binds to a neo-epitope on the same collagen type II fragment.

US Pat. No. 6642007 (Saltarelli) uses two antibodies in a sandwich, however, none of them are required to bind specifically to neo-epitopes. The capture antibody is requested to bind to type II collagen fragments, to the substantial exclusion of any binding to type I or III collagen fragments, and the other antibody, i.e. the detector antibody, should bind specifically to collagenase-generated collagen fragments. Specificity to the amino acid sequence in the cleavage site, i.e. the neo-epitope, is, however, not required.

Also, US Pat. No. 7,195,883 (Rosenquist) discloses sandwich immunoassays for type II collagen, however using a single antibody. According to the present invention, two antibodies with different epitope-specificity are required, i.e. one for each neo-epitope.

Abstract 110 in Osteoarthritis and Cartilage Vol. 16, Supplement 4, 1 September 2008, page S60, Byrjalsen et al, discloses immunoassay measurements in bovine explant cultures of two neo-epitope markers of collagen type II.

The present invention now provides a method of assay, comprising measuring in a biological sample fragments of a protein that contain an N-terminal neo-epitope and a C-terminal neo-epitope, each said neo-epitope being an amino acid sequence generated by protease cleavage of said protein, said method comprising binding the N-terminal neo-epitope with a first immunological binding partner specific for the presence of said N-terminal neo-epitope and binding the C-terminal neo-epitope with a second immunological binding partner specific for the presence of said C-terminal neo-epitope, wherein neither said first nor said second immunological binding partner specifically binds the intact protein from which said fragments derive and neither said first nor said second immunological binding partner specifically binds fragments of said protein containing the amino acid sequence of its respective said neo-epitope extended beyond the protease cleavage site, and detecting the extent of dual binding of said binding partners.

The biological sample may in particular be a body fluid sample and may be blood, serum, plasma, or urine.

Said assay may be performed as a sandwich assay in which one of said immunological binding partners is immobilised to a solid support, said fragments are bound to said immobilised antibody and the binding of the other of said immunological binding partners to said fragments is detected. The sandwich assay may be performed as a homogeneous sandwich assay or as a heterogeneous sandwich assay.

Neither said first nor said second immunological binding partner specifically binds the intact protein from which said fragments derive and preferably neither said first nor said second immunological binding partner specifically binds fragments of said protein containing the amino acid sequence of its respective said neo-epitope extended beyond the protease cleavage site.

Preferably, said neo-epitopes are from collagen type II, whereby the assay may be diagnostic of cartilage degeneration and may provide a diagnostic index in respect of arthritis.

Said neo-epitopes may be produced by cleavage of collagen type II by an MMP or by cathepsin K.

Preferably, the first immunological binding partner is specific for an epitope defined by one of the following collagen type II cleavage amino acid sequences: ⇓DQGVPG.... SEQ ID NO:54; ⇓REGSPG.... SEQ ID NO:55; *LAGPKG.... SEQ ID NO:56; and *⇓LTGPAG.... SEQ ID NO:57.

Preferably, the second immunological binding partner is specific for an epitope defined by one of the following collagen type II cleavage amino acid sequences: ....PKGARG⇓ SEQ ID NO:58; ....GQPGPA⇓ SEQ ID NO:59; ... EPGGVG⇓ SEQ ID NO:60; and ....RDGAAG⇓ SEQ ID NO:61.

Preferably, said first and second immunological binding partners are in combination specifically reactive with a collagen type II peptide fragment of the sequence:
LTGPAGEPGREGSPGADGPPGRDGAAG SEQ ID NO:62
or
REGSPGADGPPGRDGAAG SEQ ID NO:63

Optionally, severity of arthritis development in the subject is evaluated by comparing the level of binding measured in said assay with levels previously established in healthy subjects and or in subjects having pathological arthritis activity.

Assay formats useful in accordance with the present invention include both heterogeneous and homogeneous sandwich assay formats.

Homogeneous formats include the use of two different immunological binding partners bound to respective beads wherein the beads incorporate a detectable proximity signal activated when the beads are brought into proximity by their respective binding partners both binding to sites on a single fragment molecule.

Heterogeneous assay formats include those in which one of said immunological binding partners is immobilised to a solid support, said fragments are bound to said immobilised antibody and the binding of the other of said immunological binding partners to said fragments is detected.

Immunological binding partners for use in the present invention include whole antibodies, especially monoclonal antibodies, and antibody fragments with specific binding affinity. These include binding fragments such as Fab or F(ab')₂.

Assays according to the invention are useful in diagnosis or disease progression monitoring in respect of pathological conditions which lead to the release of fragments detectable in said assays. The disease indicated will depend on the fragments measured. Of especial interest for diagnosis or monitoring of osteoarthritis is collagen II. Other diseases involving destruction of cartilage could also be diagnosed and monitored with the tests according to the present invention. Such disease include rheumatoid arthritis.

Examples of neoepitopes of proteolytic cleavage of collagen type II include Cathepsin K generated neoepitopes and MMP-generated neoepitopes. Also, included are fragments generated by proteolytic cleavage of type II collagen by aggrecanases, e.g. ADAM-TS4 and ADAM-TS5. The identification of collagen type II sequences carrying Cathepsin K and MMP9 neopitopes could be performed as described below. In a similar manner collagen type II sequences carrying other MMP or aggrecanase generated neoepitopes could be identified, or MMP generated neoepitopes could be based on publicly available information on MMP generated peptide products of human articular cartilage (Zhen et al. Arthritis Rheum 2008 58(8):2420-31). To ascertain protein and protease specificity, preferred biomarker neoepitopes of cleavage sites may be selected as follows.

Human collagen type II (BIOCOL BC-3001) was dissolved in 10 mM acetic acid (400 µl added to 1 mg of collagen type II). Ten µg of procathepsin K (Calbiochem 342001) was activated by addition of 200 µl of 100 mM sodium acetate containing 10 mM DTT and 5 mM EDTA, pH 3.9 for 40 minutes at room temperature. Ten µg of MMP9 (Calbiochem 444231) was activated by addition of 200 µl of 1 mM APMA in DMSO for 2 hours at 37° C. For the Cathepsin K cleavage, 60 µl of collagen type II was added 120 µl of 50 mM sodium acetate, pH 5.5 containing 20 mM L-cystein and 24 µl of activated cathepsin K for 4 hours at 37° C. For the MMP9 cleavage, 60 µl of collagen type II was added 120 µl of 100 mM Tris-HCl, 100 MM sodium chloride, 10 mM calcium chloride, 2 mM zinc chloride, pH 8.0 and 20 µl of MMP9 for 3 days at 37° C. The resulting proteolytic cleavage fragments were characterized by high performance liquid chromatography (HPLC)-tandem mass spectrometry (MS/MS) analysis. The MS/MS spectra were searched against protein databases using Sequest and X! Tandem database search algorithms. The following sequence hits of fragments were found for the Cathepsin K cleaved collagen type II:
*AQGPPGATGFPGAAGR* SEQ ID NO:64
*ASGDRGPPGPV* SEQ ID NO:65
*ASGDRGPPGPVGPPG* SEQ ID NO:66
*GANGEKGEVGPPGPA* SEQ ID NO:67
*GAPGEDGRPGPPGPQ* SEQ ID NO:68
*GARGAPGERGETGPPGPA* SEQ ID NO:69
*GDRGPPGPV* SEQ ID NO:70
*GERGFPG* SEQ ID NO:71
*GERGFPGER* SEQ ID NO:72
*GESGSPGENGSPGPM* SEQ ID NO:73
*GLPGPPGPPGEGGKPG* SEQ ID NO:74
*GPIGPPGPA* SEQ ID NO:75
*GPPGPPGKPGDDGEAGKPG* SEQ ID NO:76
*GPPGPV* SEQ ID NO:77
*GPPGPVGPA* SEQ ID NO:78
*LPGPPGPPGEGGKPG* SEQ ID NO:79
*NPGPPGPPGPPGPG* SEQ ID NO:80
*PIGPP* SEQ ID NO:81
*REGSPGADGPPGRDGAAGVK* SEQ ID NO:82
*SNGNPGPPGPPGPS* SEQ ID NO:83

Identified Cathepsin K-generated fragments were aligned with the sequence for human collagen type II (sp|P02458|CO2A1_HUAN Collagen alpha-1(II) chain), and cleavage sites were localized as indicated by the arrows.

The following sequence hits of fragments were found for the MMP9 cleaved collagen type II:
*AAGARGNDGQPGPAGPPGPVGPA* SEQ ID NO:85
*AQGPRGEPGTPGSPGPAG* SEQ ID NO:86
*ARGAPGERGETGPPGPAG* SEQ ID NO:87
*ASGDRGPPGPV* SEQ ID NO:88
*ASGDRGPPGPVG* SEQ ID NO:89
*3ATGPLGPKG* SEQ ID NO:90
*DRGPPGPVGPPG* SEQ ID NO:91
*ERGAPGNRGFPGQDGLAGPKGAPGERGPSG* SEQ ID NO:92
*FQGLPGPPGPPGEGGKPGDQGVPGEAGAPGLVGPR* SEQ ID NO:93
*FQGLPGPPGPPGEGGKPGDQGVPGEAGAPGLVGPRG* SEQ ID NO:94
*FTGLQGLPGPPGPSG* SEQ ID NO:95
*FTGLQGLPGPPGPSGDQGASGPAGPSGPRGPPGPVGPSG* SEQ ID NO:96
*GANGEKGEVGPPGPA* SEQ ID NO:97
*GAPGEDGRPGPPGPQ* SEQ ID NO:98
*GAPGEDGRPGPPGPQG* SEQ ID NO:99
*GAPGERGETGPPGPA* SEQ ID NO:100
*GESGSPGENGSPGPM* SEQ ID NO:101
*GPIGPPGPA* SEQ ID NO:102
*GPPGPV* SEQ ID NO:103
*GPPGPVGPPG* SEQ ID NO:104
*GPRGPPGPAGAPGPQG* SEQ ID NO:105
*GVMQGPMGPMGPRGPPGPAGAPGPQG* SEQ ID NO:106
*IVGLPGQRGERGFPGLPGPSGEPGK* SEQ ID NO:107
*KQGDRGEAGAQGPMGPSGPAG* SEQ ID NO:108
*KVGPSGAPGEDGRPGPPGPQG* SEQ ID NO:109
*LPGKDGETGAAGPPGPAGPAG* SEQ ID NO:110
*LPGKDGETGAAGPPGPAGPAGERGEQGAPGPSG* SEQ ID NO:111
*LQGLPGPPGPSGDQGASGPAGPSGPRGPPGPVGPSG* SEQ ID NO:112
*LTGPAGEPGREGSPGAD* SEQ ID NO:113
*LTGPAGEPGREGSPGADGPPGRDGAAG* SEQ ID NO:62
*LTGPIGPPGPAG* SEQ ID NO:115
*LTGRPGDAGPQGKVGPSGAPGEDGRPGPPGPQG* SEQ ID NO:116
*QGPMGPMGPRGPPGPAGAPGPQG* SEQ ID NO:117
*QGPRGLPGTPGTDGPKGASGPAGPPGAQGPP* SEQ ID NO:118
*RSGETGPAGPPGNPGPPGPPGPPGPGID* SEQ ID NO:119
*RVGPPGSNGNPGPPGPPGPSG* SEQ ID NO:120
*SNGNPGPPGPPGPS* SEQ ID NO:121
*SPGPMGPRG* SEQ ID NO:122
*VKGESGSPGENGSPGPMGPRG* SEQ ID NO:123

Identified MMP9-generated fragments were aligned with the sequence for human collagen type II (sp|P02458|CO2A1_HUAN Collagen alpha-1(II) chain), and cleavage sites were localized as indicated by the stars. Cleavage sites were localized as indicated by arrows for the Cathepsin K-generated and stars for MMP9-generated sites.

Additional neoepitopes of proteolytic cleavage of collagen type II could be based on publicly available information on protease generated peptide products of human articular cartilage (Zhen et al. Arthritis Rheum 2008 58(8):2420-31).

Any of the possible generated fragments could constitute a biomarker. The biomarker could have cleavage sites at the N-terminal and the C-terminal end of the fragment and no other cleavage sites in between or the biomarker could have cleavage sites at the N-terminal and the C-terminal end and one or more possible cleavage sites in between.

Preferred type II collagen sequences carrying two neoepitopes have been selected as follows.

Preferred biomarker neoepitopes were selected based on protein specificity. The protein specificity of the cleavage sites were assessed by identity search of 6 amino-terminal or 6 carboxy-terminal residues on either site of the cleavage site. The public available programme "Pattinprot" was used in the search of the UNIPROT/SWISSPROT databank. The preferred biomarker neoepitopes of Cathepsin K cleavage sites (arrows) and/or MMP cleavage sites (stars) are indicated by underlined sequences:

An additional requirement for preferred biomarker fragments is localization of cleavage sites in proximity of each other, i.e. target fragment should preferably at maximum have a length of 50 amino acid residues, more preferably no more than 40, more preferably no more than 30. Examples of preferred biomarker fragments are:
1 LTGPAGEPGREGSPGADGPPGRDGAAG SEQ ID NO:62
2 REGSPGADGPPGRDGAAG SEQ ID NO:63

For fragment 1, the immunological binding partners should preferably have specific binding affinities for the N-terminal neoepitope of LTGPAG... SEQ ID NO:57 and the C-terminal neoepitope of ...RDGAAG SEQ ID NO:61.

For fragment 2, the immunological binding partners should preferably have specific binding affinities for the N-terminal neoepitope of REGSPG... SEQ ID NO:55 and the C-terminal neoepitope of ...RDGAAG SEQ ID NO:61.

Relevant fragments of other proteins may be defined in a similar way. The invention may be applied to the detection in biological samples of degradation products of other proteins.

The invention will be further described and illustrated by the following examples making reference to the accompanying drawings, in which:
Figure 1 shows antibody binding in sera studied in Example 1; and
Figure 2 shows monoclonal antibody binding observed in Example 1.

### Example 1

### Generation of monoclonal antibodies recognising neoepitopes

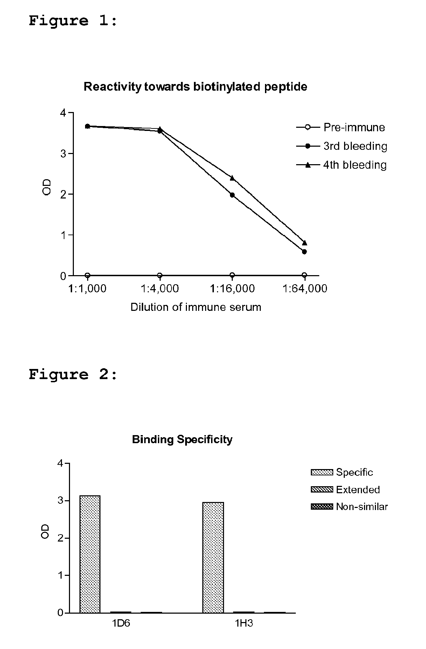

Synthetic peptides were prepared by standard techniques. To increase immunogenicity, the peptide (LTGPAGGGGC SEQ ID NO:124) was coupled at the C-terminus to the carrier protein KLH using site-directed coupling technology via the cysteine. Before immunisation, the immunogen was mixed 1:1 with Freund's Incomplete Adjuvant and the mixture was injected s.c. in Balb/c mice. The immunisation was repeated every 2 weeks for two months (four immunisations) and then continued with 4 weeks between each immunisation. Blood was obtained from the mice before immunisation initiated and one week after each immunization. The immune response was evaluated by testing the binding reactivity of mouse immune sera towards the C-terminal biotinylated synthetic peptide (LTGPAGEPGK-Biotin SEQ ID NO:125). The test for binding reactivity of mouse immune sera was based on binding of the immune serum to the biotinylated synthetic peptide that was bound to the surface of a streptavidin-coated microtitre plate. After incubation and washing the bound antibody was demonstrated by incubation with anti-mouse IgG conjugated to horseradish peroxidase, washing and addition of the chromogen TMB(Figure 1).

Subsequent to attaining sufficient immune sera titers in the above mentioned screening test, the selected mice were rested for at least 4 weeks, and boosted i.p. with immunogen without adjuvants. Three days later, the spleen was removed and used for fusion with myeloma cells using standard techniques. Antibodies from growing hybridomas were evaluated by their binding reactivity to the biotinylated synthetic peptide in the assay as described above. Additionally the cleavage specificity of the antibodies was demonstrated by minimum binding reactivity towards a one-residue extended coater (GLTGPAGEPGK-Biotin SEQ ID NO:126) as well as no binding towards a non-similar coater (Biotin-KGATGPLGPK SEQ ID NO:127)(Figure 2).

### Example 2

### Development of immunoassay for detection of collagen fragments carrying two neo-epitopes

One monoclonal antibody binding to the N-terminus of the amino acid sequence LTGPAGGGGC SEQ ID NO:124 as described above was biotinylated according to standard procedures and used as capture antibody in a sandwich ELISA. The detector antibody, binding to the C-terminus of the amino acid sequence CGGGRDGAAG SEQ ID NO:128 was labelled with horseradish peroxidase according to standard techniques. Using these two reagents and a microtitre plate coated with streptavidin, the antibodies was pre-diluted in a 10 mM phosphate buffered solution (PBS) with bovine serum albumin (1%) and Tween 20 (0.1%) in a total volume of 100 µL and subsequently incubated in the microtitre plate with 50 µL of human serum sample. A synthetic peptide with the amino acid sequence LTGPAGEPGREGSPGADGPPGRDGAAG SEQ ID NO:62 containing both the N-terminal and C-terminal neo-epitope was used as calibrator. After two hours of incubation the wells were washed and incubated with a chromogen (TMB) for 15 minutes. Subsequently, the colour reaction was stopped by addition of 0.18M sulfuric acid.

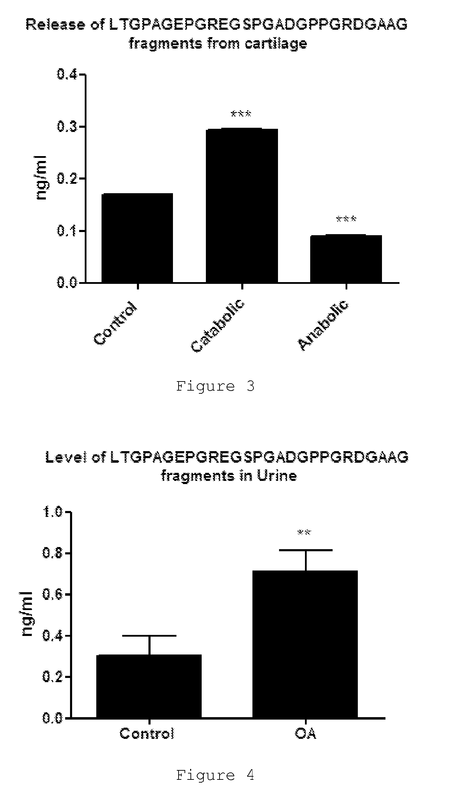

The native reactivity of the assay was tested by measurement of the release of the fragment from human articular cartilage cultured in catabolic and anabolic conditions. Figure 3 shows that catabolic condition increases the release of fragments from the cartilage whereas the anabolic condition decreases the release of the fragments. This indicates that this fragment is a marker of cartilage turnover.

Measurement of 20 human urine samples originating from patients with OA and 20 samples from healthy subjects in the ELISA described above demonstrated an increase of >50% (p<0.05) in the concentration of the collagen type II fragments in the diseased subjects (Figure 4).

### Example 3

### Development of immunoassay for detection of collagen fragments carrying one neo-epitopes - test of the whether this is a genuine MMP cleavage site.

One monoclonal antibody binding to the N-terminus of the amino acid sequence LTGPAGGGGC SEQ ID NO:124 as described above was peroxidase labelled according to standard procedures and used a biotinylated coater peptide in a competitive ELISA.
The coater peptide (LTGPAGGGGC-biotin SEQ ID NO:124 )and antibody were pre-diluted in a 10 mM phosphate buffered solution (PBS) with bovine serum albumin (1%) and Tween 20 (0.1%) in a total volume of 100 µL and subsequently incubated in the microtitre plate with 20 µL of human serum sample. A synthetic peptide with the amino acid sequence LTGPAGEPGREGSPGADGPPGRDGAAG SEQ ID NO:62 containing both the N-terminal and C-terminal neo-epitope was used as calibrator. After two hours of incubation the wells were washed and incubated with a chromogen (TMB) for 15 minutes. Subsequently, the colour reaction was stopped by addition of 0.18M sulfuric acid.

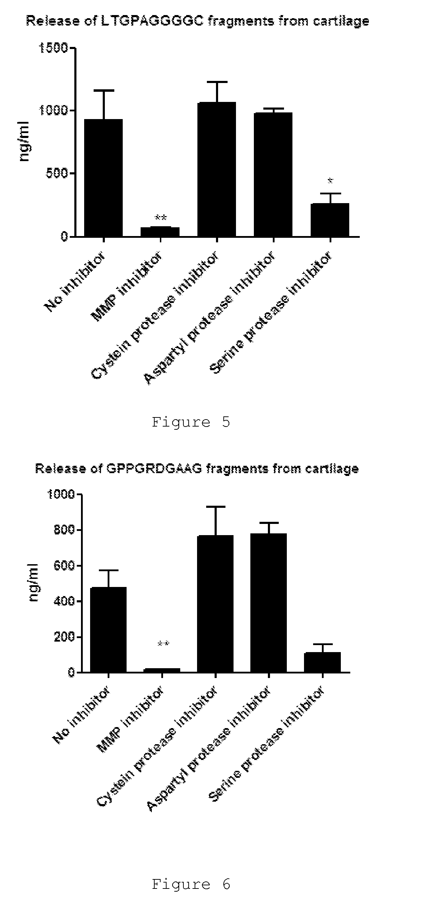

To test whether this was actually MMP-derived, different inhibitors were added to cartilage explant cultures and the release of the fragment (LTGPAGGGGC SEQ ID NO:124) to the media was measured. Figure 5 shows that the effect on the level when adding the protease inhibitors. The inhibitors used were following: metalloproteinase inhibitor (GM6001), cysteine protease inhibitor (E64), Aspartyl peptidase inhibitor Pepstatin A (Pep. A) and Serine protease inhibitor Aprotenin (apro.). Release was stimulated by adding catabolic cytokines to all conditions. Only GM6001 could block the release of the fragments, indicating that this a true MMP-derived fragment whilst the serine protease inhibitor, which indirectly inhibits MMPs, could also to a degree inhibit the release (Figure 5). This confirms that the fragment is truly a MMP-derived fragment.

### Example 4

### Development of immunoassay for detection of collagen fragments carrying one neo-epitopes - test of the whether this is a genuine MMP cleavage site.

One monoclonal antibody binding to the C-terminus of the amino acid sequence GPPGRDGAAG SEQ ID NO:114 as described above was peroxidise labelled according to standard procedures and used a biotinylated coater peptide in a competitive ELISA.
The coater peptide (biotin-GPPGRDGAAG SEQ ID NO:114)and antibody were pre-diluted in a 10 mM phosphate buffered solution (PBS) with bovine serum albumin (1%) and Tween 20 (0.1%) in a total volume of 100 µL and subsequently incubated in the micro-titre plate with 20 µL of human serum sample. A synthetic peptide with the amino acid sequence LTGPAGEPGREGSPGADGPPGRDGAAG SEQ ID NO:62 containing both the N-terminal and C-terminal neo-epitope was used as calibrator. After two hours of incubation the wells were washed and incubated with a chromogen (TMB) for 15 minutes. Subsequently, the colour reaction was stopped by addition of 0.18M sulfuric acid.

To test whether this fragment was actually MMP-derived, different inhibitors were add to cartilage explants cultures and the release of the fragment (GPPGRDGAAG SEQ ID NO:114) to the media was measured. Figure 6 shows that the effect on the level when adding the protease inhibitors. The inhibitors used were following: metalloproteinase inhibitor (GM6001), cysteine protease inhibitor (E64), Aspartyl peptidase inhibitor Pepstatin A (Pep. A) and Serine protease inhibitor Aprotenin (apro.). Release was stimulated by adding catabolic cytokines to all conditions. Only GM6001 could block the release of the fragments, indicating that this a true MMP-derived fragment. The serine protease inhibitor, which indirectly inhibits MMPs, could also to some extent inhibit the release, although not significantly(figure 6). This confirms that the fragment is truly a MMP-derived fragment.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. All prior teachings acknowledged above are hereby incorporated by reference. No acknowledgement of any prior published document herein should be taken to be an admission or representation that the teaching thereof was common general knowledge in Australia or elsewhere at the date hereof.

### References

Zhen EY, Brittain IJ, Laska DA, Mitchell PG, Sumer EU, Karsdal MA, Duffin KL. Characterization of metalloprotease cleavage products of human articular cartilage. Arthritis Rheum. 2008 Aug;58(8):2420-31
Kafienah W, Brömme D, Buttle DJ, Croucher LJ, Hollander AP. Human cathepsin K cleaves native type I and II collagens at the N-terminal end of the triple helix. Biochem J. 1998 May 1;331 (Pt 3):727-32
Dejica VM, Mort JS, Laverty S, Percival MD, Antoniou J, Zukor DJ, Poole AR. Cleavage of type II collagen by cathepsin K in human osteoarthritic cartilage. Am J Pathol. 2008 Jul;173(1):161-9. Epub 2008 May 29
Dean,D.D., Martel-Pelletier,J., Pelletier,J.P., Howell,D.S., and Woessner,J.F., Jr. 1989. Evidence for metalloproteinase and metalloproteinase inhibitor imbalance in human osteoarthritic cartilage. J Clin Invest 84:678-685.
Reboul,P., Pelletier,J.P., Tardif,G., Cloutier,J.M., and Martel-Pelletier,J. 1996. The new collagenase, collagenase-3, is expressed and synthesized by human chondrocytes but not by synoviocytes. A role in osteoarthritis. J Clin Invest 97:2011-2019.
Hui,W., Rowan,A.D., Richards,C.D., and Cawston,T.E. 2003. Oncostatin M in combination with tumor necrosis factor alpha induces cartilage damage and matrix metalloproteinase expression in vitro and in vivo. Arthritis Rheum 48:3404-3418.
Schaller,S., Henriksen,K., Hoegh-Andersen,P., Sondergaard,B.C., Sumer,E.U., Tanko,L.B., Qvist,P., and Karsdal,M.A. 2005. In vitro, ex vivo, and in vivo methodological approaches for studying therapeutic targets of osteoporosis and degenerative joint diseases: how biomarkers can assist? Assay. Drug Dev. Technol. 3:553-580.
Sumer,E.U., Schaller,S., Sondergaard,B.C., Tanko,L.B., and Qvist,P. 2006. Application of biomarkers in the clinical development of new drugs for chondroprotection in destructive joint diseases: a review. Biomarkers 11:485-506.
Christgau,S., Garnero,P., Fledelius,C., Moniz,C., Ensig,M., Gineyts,E., Rosenquist,C., and Qvist,P. 2001. Collagen type II C-telopeptide fragments as an index of cartilage degradation. Bone 29:209-215.
Mouritzen,U., Christgau,S., Lehmann,H.J., Tanko,L.B., and Christiansen,C. 2003. Cartilage turnover assessed with a newly developed assay measuring collagen type II degradation products: influence of age, sex, menopause, hormone replacement therapy, and body mass index. Ann. Rheum Dis 62:332-336.
Reijman,M., Hazes,J.M., Bierma-Zeinstra,S.M., Koes,B.W., Christgau,S., Christiansen,C., Uitterlinden,A.G., and Pols,H.A. 2004. A new marker for osteoarthritis: cross-sectional and longitudinal approach. Arthritis Rheum 50:2471-2478.
Woolf AD, Pfleger B: Burden of major musculoskeletal conditions. Bull World Health Organ 2003;81:646-56
Lawrence RC, Helmick CG, Arnett FC et al. Estimates of the prevalence of arthritis nad selected musculoskeletal disorders in the United States. Arthritis Rheum 1998;41:778-99.
Pritzker KP, Gay S, Jimenez SA, Ostergaard K, Pelletier JP, Revell PA, Salter D, van den Berg WB. Osteoarthritis cartilage histopathology: grading and staging. Osteoarthritis Cartilage 2006;14:13-29.
Birmingham JD, Vilim V, Kraus VB. Collagen biomarkers for arthritis applications. Biomarker Insights 2006; 2: 61-76.
Fraser A, Fearon U, Billinghurst RC, Ionescu M, Reece R, Barwick T, Emery P,Poole AR, Veale DJ. Turnover of type II collagen and aggrecan in cartilage matrix at the onset of inflammatory arthritis in humans: relationship to mediators of systemic and local inflammation. Arthritis Rheum. 2003 Nov;48(11):3085-95.
Billinghurst RC, Dahlberg L, Ionescu M, Reiner A, Bourne R, Rorabeck C, Mitchell P, Hambor J, Diekmann O, Tschesche H, Chen J, Van Wart H, Poole AR. Enhanced cleavage of type II collagen by collagenases in osteoarthritic articular cartilage. J Clin Invest. 1997 Apr 1;99(7):1534-45.
Poole AR, Ionescu M, Fitzcharles MA, Billinghurst RC. The assessment of cartilage degradation in vivo: development of an immunoassay for the measurement in body fluids of type II collagen cleaved by collagenases. J Immunol Methods. 2004 Nov;294(1-2):145-53.
Otterness IG, Downs JT, Lane C, Bliven ML, Stukenbrok H, Scampoli DN, Milici AJ,Mézes PS. Detection of collagenase-induced damage of collagen by 9A4, a monoclonal C-terminal neoepitope antibody. Matrix Biol. 1999 Aug;18(4):331-41.
Downs JT, Lane CL, Nestor NB, McLellan TJ, Kelly MA, Karam GA, Mezes PS, Pelletier JP, Otterness IG. Analysis of collagenase-cleavage of type II collagen using a neoepitope ELISA. J Immunol Methods. 2001 Jan 1;247(1-2):25-34.

## Claims

1. A method of assay, comprising measuring in a biological sample fragments of a protein that contain an N-terminal neo-epitope and a C-terminal neo-epitope, each said neo-epitope being an amino acid sequence generated by protease cleavage of said protein, said method comprising binding the N-terminal neo-epitope with a first immunological binding partner specific for the presence of said N-terminal neo-epitope and binding the C-terminal neo-epitope with a second immunological binding partner specific for the presence of said C-terminal neo-epitope, wherein neither said first nor said second immunological binding partner specifically binds the intact protein from which said fragments derive and neither said first nor said second immunological binding partner specifically binds fragments of said protein containing the amino acid sequence of its respective said neo-epitope extended beyond the protease cleavage site, and detecting the extent of dual binding of said binding partners.

2. A method as claimed in claim 1, wherein said assay is performed as a sandwich assay in which one of said immunological binding partners is immobilised to a solid support, said fragments are bound to said immobilised antibody and the binding of the other of said immunological binding partners to said fragments is detected.

3. A method as claimed in claim 1, wherein the assay is performed as a homogeneous sandwich assay.

4. A method as claimed in any preceding claim, wherein said neo-epitopes are from collagen type II.

5. A method as claimed in any preceding claim, wherein said neo-epitopes are produced by cleavage of collagen type II by an MMP or by cathepsin K.

6. A method as claimed in claim 5, wherein the first immunological binding partner is specific for an epitope defined by one of the following collagen type II cleavage amino acid sequences: ⇓DQGVPG....SEQ ID NO:54; ⇓REGSPG.... SEQ ID NO:55; *LAGPKG.... SEQ ID NO:56;and *⇓LTGPAG.... SEQ ID NO:57.

7. A method as claimed in claim 5 or claim 6, wherein the second immunological binding partner is specific for an epitope defined by one of the following collagen type II cleavage amino acid sequences:
....PKGARG⇓ SEQ ID NO:58; ....GQPGPA⇓ SEQ ID NO:59;
... EPGGVG⇓ SEQ ID NO:60; and ....RDGAAG⇓ SEQ ID NO:61.

8. A method as claimed in any preceding claim, wherein said first and second immunological binding partners are in combination specifically reactive with a collagen type II peptide fragment of the sequence:
LTGPAGEPGREGSPGADGPPGRDGAAG SEQ ID NO:62
or
REGSPGADGPPGRDGAAG SEQ ID NO:63

9. A method as claimed in claim 5, wherein said immunological binding partner does not specifically bind a sequence as defined in claim 5 if continued past the indicated cleavage site.

## Patentansprüche

1. Verfahren eines Assays, das die Messung von Fragmenten eines Proteins, die ein N-Terminus-Neo-Epitop und ein C-Terminus-Neo-Epitop umfassen, in einer biologischen Probe umfasst, wobei jedes dieser Neo-Epitope eine Aminosäuresequenz ist, die durch die Proteasespaltung dieses Proteins erzeugt wird, wobei dieses Verfahren die Bindung des N-Terminus-Neo-Epitops mit einem ersten immunologischen Bindungspartner, der spezifisch ist für das Vorhandensein dieses N-Terminus-Neo-Epitops, und die Bindung des C-Terminus-Neo-Epitops mit einem zweiten immunologischen Bindungspartner, der spezifisch ist für das Vorhandensein dieses C-Terminus-Neo-Epitops, umfasst, wobei weder der erste noch der zweite immunologische Bindungspartner spezifisch das intakte Protein bindet, von dem sich diese Fragmente ableiten, und weder der erste noch der zweite immunologische Bindungspartner spezifisch Fragmente dieses Proteins bindet, die die Aminosäuresequenz ihres jeweiligen Neo-Epitops enthalten, die sich über die Proteasespaltungsstelle hinaus erstreckt, und die Erkennung des Ausmaßes der dualen Bindung dieser Bindungspartner umfasst.

2. Verfahren gemäß Anspruch 1, wobei dieser Assay als Sandwich-Assay durchgeführt wird, in dem einer der immunologischen Bindungspartner zu einem festen Träger immobilisiert wird, die Fragmente an diesen immobilisierten Antikörper gebunden werden und die Bindung des anderen dieser immunologischen Bindungspartner an diese Fragmente erkannt wird.

3. Verfahren gemäß Anspruch 1, wobei der Assay als homogener Sandwich-Assay durchgeführt wird.

4. Verfahren gemäß einem der vorgenannten Ansprüche, wobei die Neo-Epitope vom Kollagen-Typ II sind.

5. Verfahren gemäß einem der vorgenannten Ansprüche, wobei die Neo-Epitope durch Spaltung des Kollagen-Typs II durch ein MMP oder durch Kathepsin K erzeugt werden.

6. Verfahren gemäß Anspruch 5, wobei der erste immunologische Bindungspartner spezifisch ist für ein Epitop, das durch eine der folgenden Aminosäuresequenzen der Kollagen-Typ II-Abspaltung definiert ist: ⇓DQGVPG.... SEQ.-ID-NR:54; ⇓REGSPG.... SEQ.-ID-NR:55; *LAGPKG.... SEQ.-ID-NR:56; und *⇓LTGPAG.... SEQ.-ID-NR:57.

7. Verfahren gemäß Anspruch 5 oder Anspruch 6, wobei der zweite immunologische Bindungspartner spezifisch ist für ein Epitop, das durch eine der folgenden Aminosäuresequenzen der Kollagen-Typ II-Abspaltung definiert ist:
....PKGARG⇓ SEQ.-ID-NR:58; ....GQPGPA⇓ SEQ.-ID-NR:59;
... EPGGVG↓ SEQ.-ID-NR: 60; und .... RDGAAG⇓ SEQ.-ID-NR: 61.

8. Verfahren gemäß einem der vorgenannten Ansprüche, wobei diese ersten und zweiten immunologischen Bindungspartner in Kombination insbesondere reaktionsfreudig sind mit einem Kollagen-Typ II-Peptidfragment der Sequenz:
LTGPAGEPGREGSPGADGPPGRDGAAG SEQ.-ID-NR:62
oder
REGSPGADGPPGRDGAAG SEQ.-ID-NR:63

9. Verfahren gemäß Anspruch 5, wobei dieser immunologische Bindungspartner nicht spezifisch eine Sequenz bindet, wie in Anspruch 5 definiert, wenn diese über die angegebene Spaltungsstelle hinausgeht.

## Revendications

1. Méthode de dosage, comprenant la mesure, dans un échantillon biologique, des fragments d'une protéine qui contiennent un néo-épitope N-terminal et un néo-épitope C-terminal, chaque épitope étant une séquence d'acides aminés générée par le clivage par protéase de ladite protéine, ladite méthode comprenant la liaison du néo-épitope N-terminal avec un premier partenaire de liaison immunologique spécifique de la présence dudit néo-épitope N-terminal et la liaison du néo-épitope C-terminal avec un second partenaire de liaison immunologique spécifique de la présence dudit néo-épitope C-terminal, ni ledit premier ni ledit second partenaire de liaison immunologique ne se liant spécifiquement à la protéine intacte de laquelle lesdits fragments proviennent et ni le premier ni le second partenaire de liaison immunologique ne se liant spécifiquement aux fragments de ladite protéine contenant la séquence d'acides aminés dudit néo-épitope respectif qui s'étend au-delà du site de clivage par protéase, et la détection de l'amplitude de la double liaison desdits partenaires de liaison.

2. Méthode selon la revendication 1, ledit dosage étant effectué sous forme de dosage sandwich dans lequel l'un desdits partenaires de liaison immunologiques est immobilisé sur un support solide, lesdits fragments sont liés audit anticorps immobilisé et la liaison de l'autre desdits partenaires de liaison immunologiques auxdits fragments est détectée.

3. Méthode selon la revendication 1, ledit dosage étant effectué sous la forme d'un dosage sandwich homogène.

4. Méthode selon l'une quelconque des revendications précédentes, lesdits néo-épitopes étant issus de collagène de type II.

5. Méthode selon l'une quelconque des revendications précédentes, lesdits néo-épitopes étant produits par clivage d'un collagène de type II par une MMP ou par la cathepsine K.

6. Méthode selon la revendication 5, ledit premier partenaire de liaison immunologique étant spécifique d'un épitope défini par l'une des séquences d'acides aminés suivantes obtenues par clivage d'un collagène de type II : SEQ ID n° : 54 ⇓DQGVPG.... ; SEQ ID n° : 55 ⇓REGSPG.... ; SEQ ID n° : 56 *LAGPKG.... ; et SEQ ID n° : 57 *⇓LTGPAG.....

7. Méthode selon la revendication 5 ou la revendication 6, ledit second partenaire de liaison immunologique étant spécifique d'un épitope défini par l'une des séquences d'acides aminés suivantes obtenues par clivage d'un collagène de type II :
SEQ ID n° : 58 ....PKGARG⇓ ; SEQ ID n° : 59 ....GQPGPA⇓;
SEQ ID n° : 60 ... EPGGVG⇓ ; et SEQ ID n° : 61 .... RDGAAG⇓.

8. Méthode selon l'une quelconque des revendications précédentes, lesdits premier et second partenaires de liaison immunologiques étant en combinaison spécifiquement réactive avec un fragment peptidique de collagène de type II de la séquence :
SEQ ID n° : 62 LTGPAGEPGREGSPGADGPPGRDGAAG ou
SEQ ID n° : 63 REGSPGADGPPGRDGAAG.

9. Méthode selon la revendication 5, ledit partenaire de liaison immunologique ne se liant pas spécifiquement à une séquence telle que définie selon la revendication 5 si elle continue au-delà du site de clivage indiqué.
